# EUROPÄISCHE PATENTSCHRIFT

(11) **EP 2 727 907 B1**
(45) Veröffentlichungstag und Bekanntmachung des Hinweises auf die Patenterteilung: **13.05.2015**
(21) Anmeldenummer: 12190588.9
(22) Anmeldetag: 30.10.2012
(51) Int. Cl.: C07C 323/62, C08K 5/375, C08L 13/00

(54) **Polysulfidmischung, Verfahren zu ihrer Herstellung und Verwendung der Polysulfidmischung in Kautschukmischungen**
Polysulphide mixture, process for its preparation and use of the polysulphide mixture in rubber mixtures
Mélange polysulfure, son procédé de fabrication et utilisation du mélange polysulfure dans des mélanges de caoutchouc

(43) Veröffentlichungstag der Anmeldung: 07.05.2014
(73) Patentinhaber: LANXESS Deutschland GmbH, 50569 Köln (DE)
(72) Erfinder: Feldhues, Ulrich, 51465 Bergisch Gladbach (DE); Unterberg, Heinz, 41540 Dormagen (DE); Weidenhaupt, Hermann-Josef, 50259 Pulheim (DE); Wiedemeier-Jarad, Melanie, 41540 Dormagen (DE)

(56) Entgegenhaltungen:
- EP-A1- 2 517 898
- US-A- 3 298 996

## Beschreibung

Die vorliegende Erfindung betrifft neue Polysulfidmischungen, Verfahren zur Herstellung dieser Polysulfidmischungen, die Verwendung der Polysulfidmischungen in Kautschukmischungen, daraus hergestellte Kautschukvulkanisate und deren Verwendung.

Kieselsäurehaltige Kautschukmischungen sind wichtige Ausgangsmaterialien, beispielsweise für die Herstellung von Reifen mit verringertem Rollwiderstand. Diese leisten beim Abrollen weniger Verformungsarbeit (als Reifen, welche allein Ruß als Füllstoff enthalten) und senken deshalb den Kraftstoffverbrauch. Durch die in verschiedenen Staaten beschlossene Kennzeichnungspflicht des Rollwiderstands für Reifen besteht ein großes Interesse daran, diesen Widerstand weiter zu senken.

Zur Verbesserung der physikalischen Eigenschaften der Vulkanisate werden, wie beispielsweise in DE 2 255 577 A erwähnt, polysulfidische Silane als Verstärkungsadditive eingesetzt. Das Eigenschaftsprofil der so hergestellten Kautschukvulkanisate ist noch nicht optimal. Neben einer Verbesserung des Rollwiderstands ist insbesondere eine niedrige Fließviskosität (Mooney Viskosität ML 1+4/100°C) der Kautschukmischung erwünscht, was die Verarbeitbarkeit begünstigt. Hierfür wurden weitere Zusatzstoffe vorgeschlagen, wie Fettsäureester, Fettsäuresalze oder Mineralöle, welche zwar die Fließfähigkeit erhöhen, gleichzeitig aber die Spannungswerte bei größerer Dehnung (z.B. 100% bis 300%) oder auch die Härte der Vulkanisate vermindern, so dass die verstärkende Wirkung des Füllstoffes nicht mehr voll zum Tragen kommt. Eine zu geringe Härte oder Steifigkeit des Vulkanisats resultiert aber in unbefriedigendes Fahrverhalten des Reifens besonders in Kurven. Zur Erhöhung der Härte des Vulkanisats kann der Anteil an verstärkendem Füllstoff erhöht, oder der Anteil an Weichmacheröl verringert werden, wobei jede dieser beiden Maßnahmen den Nachteil der höheren Mischungsviskosität bei der Verarbeitung bedingt.

In EP 0 489 313 werden Glykolfunktionen enthaltende Additive mit guten mechanischen Eigenschaften und verbessertem Hystereseverhalten beschrieben. Im Vergleich mit dem Bis-[3-(triethoxysilyl)-propyl]-tetrasulfid gemäß DE-OS 2 255 577 zeigen die Beispiele aber keine Verbesserung des Rollwiderstands (tan δ bei 60°C).

Dokument US3298996 beschreibt Verbindungen mit m = 0 und ihre Verwendung zur Stabilisierung von Olefinpolymeren. Ein spätes Dokument (EP2517898) beschreibt Verbindungen mit m = 0 und m = 1 , aber keine Zusammensetzungen mit den zwei Verbindungen.

In EP 1 000 968 konnte eine Verbesserung der physikalischen Eigenschaften durch Verwendung eines polysulfidischen Silans in Kombination mit einem speziellen Reversionsschutzmittel in SBR erzielt werden, wobei aber weder bei der Mischungs-viskosität noch beim Rollwiderstand (tan δ bei 60°C) eine signifikante Änderung gegenüber dem Stand der Technik erzielt wurde.

In der bis dato unveröffentlichten Anmeldung EP 11164319 ist die Verwendung einer Verbindung mit der idealisierten Struktur (II) beschrieben. Für den Fachmann auf dem Gebiet der Polysulfidverbindungen ist - in Anbetracht der schwierigen Auftrennung von Polysulfiden - anhand der Formulierung in der EP 11164319 offensichtlich, dass es sich um eine Mischung verschiedener Verbindungen handelt, welche gemittelt über die Anzahl Schwefelatome und Alkylenbrücken ungefähr der Formel (II) entspricht. Eine Analyse der Zusammensetzung der Mischung wurde nicht durchgeführt.

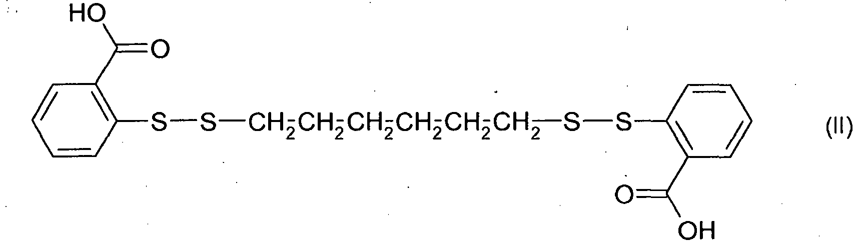

Gegenüber einer Referenzmischung ohne die Verbindung mit der idealisierten Struktur (II) wurden Abrieb und Rollwiderstand verschlechtert. Zudem stieg die Anvulkanisationsdauer nach Mooney bei 130°C unerwünscht hoch an.

Aufgabe der vorliegenden Erfindung ist die Bereitstellung neuer Kautschukadditive, Verfahren zu deren Herstellung und neuer Kautschukmischungen, welche bei gleicher oder verbesserter Fließfähigkeit der Kautschukmischungen in Vulkanisate mit vermindertem Rollwiderstand überführt werden können, bei denen aber zugleich die ebenfalls wichtigen Parameter Shore A Härte, Modul 300, Bruchdehnung, Zugfestigkeit und Abrieb keine wesentliche Beeinträchtigung zeigen.

Die Nacharbeitung der in EP 11164319 offenbarten Mischung zeigte, dass diese neben 54% der Verbindung der Formel (II) 25% 2,2'-Dithiobisbenzoesäure und 21% einer Verbindung enthält, bei der die beiden Benzoesäurereste jeweils in 2-Position mittels einer S₂-(CH₂)₆-S₂-(CH₂)₆-S₂ Kette verbunden sind.

Überraschend wurde nun gefunden, dass bei einer engeren Verteilung der S-(CH₂)₆-S-Einheiten in der Polysulfidmischung das Eigenschaftsprofil von Kautschukmischungen enthaltend diese Polysulfidmischung und deren Vulkanisat deutlich verbessert wird und dadurch die erfindungsgemäße Aufgabe gelöst wird.

Zudem wurde gefunden, dass nicht nur die Verbindungen mit der idealisierten Struktur (II) die gewünschten Eigenschaften der Kautschukmischungen und daraus gewonnener Vulkanisate erzielen, sondern auch Salze dieser Verbindungen, insbesondere Zinksalze.

Die vorliegende Erfindung betrifft daher Polysulfidmischungen enthaltend zwei oder mehr Verbindungen der Formel (I), 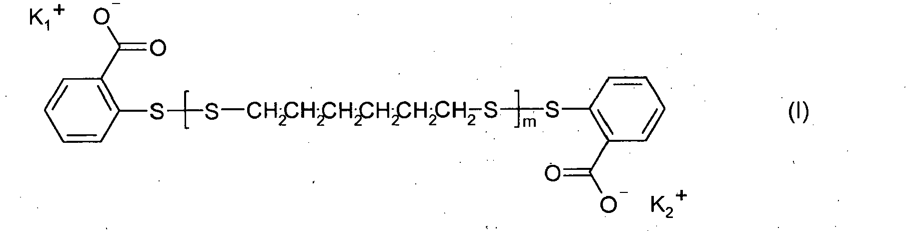 wobei die Kationen K₁⁺ und K₂⁺ unabhängig voneinander für beliebige einwertige oder für den n-ten Teil eines beliebigen n-wertigen Kations stehen und m für 0, 1 und/oder 2 steht, dadurch gekennzeichnet, dass der gesamte Anteil an Verbindung(en) mit m = 1 wenigstens 80%, bevorzugt wenigstens 85%, besonders bevorzugt wenigstens 90%, ganz besonders bevorzugt 93 bis 99% beträgt, bezogen auf die Gesamtmenge an Polysulfidverbindungen der Formel (I). Im Rahmen der vorliegenden Anmeldung sind die Mengenangaben in Bezug auf die Verbindungen der Formel (I) als Flächenprozentangaben bei der HPLC-Messung mit UV-Detektor zu verstehen, wie sie im Anschluss an Beispiel 3 beschrieben ist.

Vorzugsweise stehen die Kationen K₁⁺ und K₂⁺ unabhängig voneinander für H⁺, für ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺.

Ganz besonders bevorzugt stehen die Kationen K₁⁺ und K₂⁺ unabhängig voneinander für H⁺ oder für ½ Zn²⁺, insbesondere für ½ Zn²⁺.

Typischerweise enthalten erfindungsgemäße Polysulfidmischungen insgesamt bis zu 10%, bevorzugt bis zu 7,5%, besonders bevorzugt bis zu 5% und ganz besonders bevorzugt nicht mehr als 3% an Polysulfidverbindungen der Formel (I) mit m = 0, bezogen auf die Gesamtmenge an Polysulfidverbindungen der Formel (I).

Entsprechend enthalten erfindungsgemäße Polysulfidmischungen gewöhnlich insgesamt bis zu 10%, bevorzugt bis zu 7,5%, besonders bevorzugt bis zu 5% und ganz besonders bevorzugt weniger als 2% an Polysulfidverbindungen der Formel (I) mit m = 2, bezogen auf die Gesamtmenge an Polysulfidverbindungen der Formel (I).

Der gesamte Anteil an Polysulfiden der Formel (I) mit m = 0 und/oder m = 2 in den erfindungsgemäßen Polysulfidmischungen beträgt bis zu 20%, vorzugsweise bis zu 15%, besonders bevorzugt bis zu 10% und ganz besonders bevorzugt 1 bis 7% bezogen auf die Gesamtmenge an Polysulfidverbindungen der Formel (I).

Im Regelfall weisen erfindungsgemäße Polysulfidmischungen einen Schwefelgehalt gemäß Elementaranalyse von 22 - 32%, bevorzugt von 24 - 30% und besonders bevorzugt von 27 - 29% auf.

In den erfindungsgemäßen Polysulfidmischungen besitzen die Moleküle der Formel (I) normalerweise im Durchschnitt 3,5 - 4,5, bevorzugt 3,7 - 4,3, besonders bevorzugt 3,8 - 4,2 und ganz besonders bevorzugt 3,9 - 4,1 Schwefelatome.

In einer bevorzugten Ausführungsform enthält die erfindungsgemäße Polysulfidmischung weniger als 10%, besonders bevorzugt weniger als 3%, ganz besonders bevorzugt weniger als 1% Nebenprodukte oder Beimischungen, also Verbindungen, die nicht der Formel (I) entsprechen.

Typischerweise enthält die erfindungsgemäße Polysulfidmischung weniger als 2%, bevorzugt weniger als 1%, besonders bevorzugt weniger als 0,3%, ganz besonders bevorzugt weniger 0,1% und meist bevorzugt keinen elementaren Schwefel als Beimischung.

Typischerweise enthält die erfindungsgemäße Polysulfidmischung weniger als 2%, bevorzugt weniger als 1%, besonders bevorzugt weniger als 0,3%, ganz besonders bevorzugt weniger 0,1% und meist bevorzugt keine Beschleuniger der Mercapto- bzw. Sulfenamid-Gruppe als Beimischung.

Eine erfindungsgemäße Polysulfidmischung hat typischerweise einen Gesamt-ChlorGehalt < 1%, bevorzugt < 1000ppm, besonders bevorzugt < 200ppm, ganz besonders bevorzugt < 50ppm, am meisten bevorzugt < 10ppm.

Die vorliegende Erfindung umfasst auch ein Verfahren zur Herstellung der erfindungsgemäßen Polysulfidmischungen durch Inkontaktbringen von 2-Mercaptobenzoesäure oder deren Salzen (III) mit Hexamethylen-1,6-bisthiosulfaten (IV).

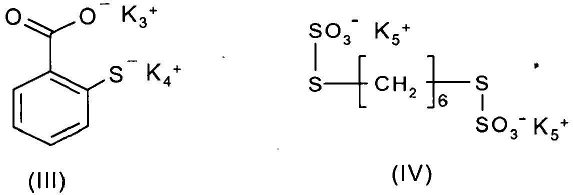

Hierbei stehen die Kationen K₃⁺ und K₄⁺ unabhängig voneinander für beliebige einwertige Kationen oder für den n-ten Teil eines beliebigen n-wertigen Kations, vorzugsweise für H⁺, für ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺, besonders bevorzugt für H⁺, Na⁺, K⁺ oder für ½ Zn²⁺. Die beiden Kationen K₅⁺ sind dabei gleich oder verschieden, vorzugsweise gleich und stehen unabhängig voneinander für beliebige einwertige Kationen oder für den n-ten Teil eines beliebigen n-wertigen Kations, vorzugsweise für ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺, besonders bevorzugt für Na⁺.

In einer besonders bevorzugten Form wird als Verbindung der Formel (IV) das kommerziell erhältliche Dinatriumhexamethylen-1,6-bisthiosulfatdihydrat eingesetzt.

Das Inkontaktbringen von Verbindung(en) der Formel (III) mit Verbindung(en) der Formel (IV) findet üblicherweise in einem wässrigen oder wässrig-organischen Medium statt, wobei eine Schutzgasatmosphäre vorteilhaft ist, um Oxidationsprodukte zu verhindern.

Das wässrig-organische Medium enthält dabei Wasser und ein oder mehrere organische Lösungsmittel, insbesondere Lösungsmittel aus der Gruppe Alkohole, Ester und Ether.

Als Schutzgase können alle Gase verwendet werden, welche unter Reaktionsbedingungen nicht oder nur unwesentlich reagieren. Vorzugsweise werden Edelgase oder Stickstoff verwendet.

In einer bevorzugten Ausführungsform geschieht das Inkontaktbringen in Gegenwart von Aldehyden und/oder Ketonen, insbesondere in Gegenwart von Formaldehyd.

Das Inkontaktbringen erfolgt vorzugsweise bei Temperaturen im Bereich von -5°C bis 19°C, besonders bevorzugt im Bereich von 0°C bis 15°C, ganz besonders bevorzugt im Bereich von 0°C bis 10°C. Zu hohe Temperaturen und lange Reaktions- und Nachrührzeiten führen vermehrt zu Nebenprodukten. Bei Temperaturen im Bereich von 0 - 10°C können mit sehr hoher Selektivität Verbindungen der Formel (I) mit m = 1 erzeugt werden. Bereits bei Temperaturen ≥ 20°C verringert sich die Selektivität für diese Substanz und insbesondere bei längeren Reaktionszeiten nimmt der Anteil an Produkten der Formel (I) mit m = 1 ab, wobei der Anteil an Verbindungen der Formel (I) mit m = 0 ansteigt, welche aber beim Einsatz in Kautschuk nicht zur gewünschten Verringerung des Rollwiderstands des Vulkanisats führt.

In einer bevorzugten Ausführungsform wird das wässrige Medium, Verbindung(en) (IV) und Formaldehyd vorgelegt und 2-Mercaptobenzoesäure (III) als wässrige Lösung ihrer Salze, d. h. Verbindung(en) der Formel (III), wobei K₃⁺ und/oder K₄⁺ nicht für H⁺ stehen, zudosiert, wobei der pH im Bereich von 7-13, besonders bevorzugt 8-12, insbesondere 9-11 gehalten wird, vorzugsweise durch Zugabe einer Brönsted-Säure. Diese kann in beliebiger Konzentration, vorzugsweise jedoch verdünnt, zugegeben werden. Besonders bevorzugt wird eine oder mehrere Mineralsäuren verwendet. Durch anschließende Zugabe von weiterer Säure wird der pH vorzugsweise auf einen Bereich von 0-4, insbesondere 1 - 3 gestellt. Dadurch können die Verbindungen der Formel (V), in denen m für 0, 1 und/oder 2 steht, ausgefällt und erfindungsgemäße Polysulfidmischungen auf Basis von Polysulfiden der Formel (I) erhalten werden, bei denen K₁⁺ und K₂⁺ für H⁺ stehen.

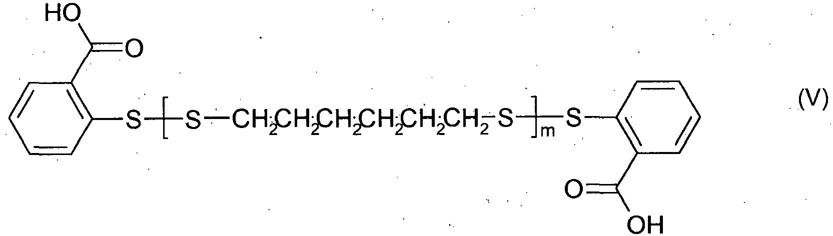

Erfindungsgemäße Polysulfide der Formel (I), in denen wenigstens eines der Kationen K₁⁺ und/oder K₂⁺ für ein anderes Kation als H⁺ stehen, können durch Inkontakbringen von Salzen dieser Kationen K₁⁺ und/oder K₂⁺ (insbesondere in Form ihrer wässrigen Lösungen) mit Polysulfiden der Formel (I) hergestellt werden. Als Salze werden hierfür vorzugsweise Sulfate, Hydrogensulfate, Phosphate, Hydrogenphosphate, Dihydrogenphosphate, Carbonate, Hydrogencarbonate, Hydroxide, Nitrate, Chloride und Acetate, besonders bevorzugt Sulfate eingesetzt.

Das Inkontaktbringen mit obigen Salzen findet bevorzugt in wässrigem Medium und bei Temperaturen von vorzugsweise 0 - 20°C, insbesondere 0 - 10°C und vorzugsweise bei pH-Werten von 3-10, bevorzugt von 4-9, insbesondere von 5-8 statt. Erfindungsgemäße Polysulfide der Formel (I), in denen K₁⁺ und/oder K₂⁺, für ein anderes Kation als H⁺ stehen, können auch ohne Zwischenisolierung von Verbindungen der Formel (V) oder deren Salze vorteilhaft in einem Eintopfverfahren hergestellt werden.

Erfindungsgemäße Polysulfide der Formel (I), in denen K₁⁺ und/oder K₂⁺, für ein anderes Kation als H⁺ stehen, werden vorzugsweise dadurch erhalten, dass nach Kontaktieren der Verbindung(en) der Formel (III) mit mindestens einer Verbindung der Formel (IV) die Polysulfide durch Inkontaktbringen mit einem Salz der Kationen K₁⁺ und/oder K₂⁺, wobei K₁⁺ und/oder K₂⁺ für ein anderes Kation als H⁺ stehen, bevorzugt mit einem Zinksalz, insbesondere mit Zinksulfat, ausgefällt werden.

Die vorliegende Erfindung betrifft daher auch Polysulfidverbindungen der Formel (I) bei denen K₁⁺ und/oder K₂⁺ vorzugsweise K₁⁺ und K₂⁺ für ein anderes Kation als H⁺, vorzugsweise für ½ Zn²⁺ stehen. Insbesondere umfasst die vorliegende Erfindung Polysulfidverbindungen der Formel (I), in denen K₁⁺ und/oder K₂⁺ für ½ Zn²⁺ und m für 1 und/oder 2 steht, insbesondere für 1 steht.

Die entsprechenden Polysulfidverbindungen der Formel (I) bei denen K₁⁺ und/oder K₂⁺ für ein anderes Kation als H⁺, vorzugsweise für ½ Zn²⁺ stehen sind u. a. durch das erfindungsgemäße Herstellungsverfahren erhältlich. Da solche Polysulfidverbindungen, insbesondere Verbindungen mit m = 1, ganz besonders bevorzugt die Verbindung mit m = 1, bei der K₁⁺ und K₂⁺ für ½ Zn²⁺ stehen, bei Verwendung in Kautschukmischungen den erfindungsgemäßen Effekt erzielen, sind sie als zu den erfindungsgemäßen Polysulfidmischungen äquivalent zu betrachten.

Mit dem vorliegenden Verfahren insbesondere in den bevorzugten Ausführungsformen, lassen sich die erfindungsgemäßen Polysulfidmischungen in sehr hoher Ausbeute herstellen.

Vorzugsweise werden die erfindungsgemäßen Polysulfidmischungen auf Basis von Verbindungen der Formel (I) nach der Herstellung bei Temperaturen zwischen 0 - 35°C gelagert.

Überraschend wurde nun gefunden, dass durch die erfindungsgemäßen Polysulfidmischungen die Fließfähigkeit von Kautschukmischungen und deren Anvulkanisationszeit verbessert wird und zugleich Vulkanisate mit geringerem Rollwiderstand und geringerem Abrieb erhalten werden.

Gegenstand der Erfindung sind daher Kautschukmischungen, enthaltend jeweils mindestens einen Kautschuk und eine erfindungsgemäße Polysulfidmischung auf Basis von Verbindungen der Formel (I).

Gegenstand der Erfindung sind insbesondere Kautschukmischungen, enthaltend jeweils mindestens einen Kautschuk, ein schwefelhaltiges Alkoxysilan, einen kieselsäurebasierenden Füllstoff, und eine erfindungsgemäße Polysulfidmischung auf Basis von Verbindungen der Formel (I). Bevorzugte Kautschukmischungen enthalten die bevorzugten Polysulfidmischungen.

Die erfindungsgemäßen Polysulfidmischungen können auch teilweise oder vollständig auf inerten, organischen oder anorganischen Trägern absorbiert eingesetzt werden. Bevorzugte Trägermaterialien sind Kieselsäure, natürliche und synthetische Silikate, Aluminiumoxid und/oder Russe.

Der Gesamtgehalt der erfindungsgemäßen Polysulfidmischung in den erfindungsgemäßen Kautschukmischungen beträgt vorzugsweise 0,1 bis 15 phr, besonders bevorzugt 0,3 bis 7 phr, ganz besonders bevorzugt 0,5 bis 3 phr und am meisten bevorzugt 0,7 bis 1,5 phr. Die Einheit phr steht für Gewichtsteile bezogen auf 100 Gewichtsteile an in der Kautschukmischung eingesetztem Kautschuk.

Für die Herstellung der erfindungsgemäßen Kautschukmischungen können Naturkautschuk und/oder Synthesekautschuke verwendet werden. Bevorzugte Synthesekautschuke sind beispielsweise

| | | |
|---|---|---|
| BR | - | Polybutadien |
| ABR | - | Butadien/Acrylsäure-C1-C4-alkylester-Copolymerisat |
| CR | - | Polychloropren |
| IR | - | Polyisopren |
| SBR | - | Styrol/Butadien-Copolymerisate mit Styrolgehalten von 1-60, bevorzugt 20-50 Gew.-% |
| IIR | - | Isobutylen/Isopren-Copolymerisate |
| NBR | - | Butadien/Acrylnitril-Copolymerisate mit 5-60, vorzugsw. 10-50 Gew-% Acrylnitrilgehalt |
| HNBR | - | teilhydrierter oder vollständig hydrierter NBR-Kautschuk |
| EPDM | - | Ethylen/Propylen/Dien-Copolymerisate |

sowie Mischungen aus zwei oder mehr dieser Kautschuke.

Bevorzugt enthalten die erfindungsgemäßen Kautschukmischungen mindestens einen SBR-Kautschuk und mindestens einen BR-Kautschuk, besonders bevorzugt im Gewichtsverhältnis SBR:BR von 60:40 bis 90:10.

In einer vorteilhaften Ausführungsform enthalten die erfindungsgemäßen Kautschukmischungen zudem mindestens einen NR-Kautschuk. Besonders bevorzugt weisen sie wenigstens einen SBR-Kautschuk, wenigstens einen BR-Kautschuk und wenigstens einen NR-Kautschuk auf, wobei ganz besonders bevorzugt das Gewichtsverhältnis von SBR-Kautschuk zu BR-Kautschuk zu NR-Kautschuk 60 bis 85:10 bis 35:5 bis 20 beträgt.

Als schwefelhaltige Alkoxysilane für die erfindungsgemäßen Kautschukmischungen sind z.B. Bis-(triethoxysilylpropyl)tetrasulfan (z.B. Si 69 von Evonik) und Bis-(triethoxysilyl-propyl)disulfan (z.B. Si 75 von Evonik), 3-(Triethoxysilyl)-1-propanthiol, polyetherfunktionalisierte Mercaptosilane wie Si 363 von Evonik, thioesterfunktionalisierte Alkoxysilane wie NXT oder NXT Z von Momentive (früher GE) geeignet. Es können auch Mischungen der schwefelhaltigen Alkoxysilane eingesetzt werden. Flüssige schwefelhaltige Alkoxysilane können zur besseren Dosierbarkeit und/oder Dispergierbarkeit auf einem Träger aufgezogen sein (dry liquid). Der Wirkstoffgehalt liegt zwischen 30 und 70 Gew.-Teilen, bevorzugt 40 und 60 Gew.-Teilen je 100 Gew.-Teile dry liquid.

Der Anteil der schwefelhaltigen Alkoxysilane in den erfindungsgemäßen Kautschukmischungen beträgt vorzugsweise 2 bis 20 phr, besonders bevorzugt 3 bis 11 phr und ganz besonders bevorzugt 5 bis 8 phr, jeweils berechnet als 100%iger Wirkstoff. Vorzugsweise ist die Menge an schwefelhaltigem Alkoxysilan größer oder gleich der Menge an der erfindungsgemäßen Polysulfidmischung auf Basis von Verbindungen der Formel (I). Besonders bevorzugt beträgt das Gewichtsverhältnis von schwefelhaltigem Alkoxysilan zur erfindungsgemäßen Polysulfidmischung auf Basis von Verbindungen der Formel (I) 1,5:1 bis 20:1, ganz besonders bevorzugt 3:1 bis 15:1 und am meisten bevorzugt 5:1 bis 10:1.

Die erfindungsgemäß bevorzugte Kautschukmischung enthält zudem einen oder mehrere kieselsäurebasierende Füllstoffe. Vorzugsweise werden dafür folgende Stoffe verwendet:
- Kieselsäure, insbesondere Fällungskieselsäure oder pyrogene Kieselsäure, hergestellt z.B. durch Fällung von Lösungen von Silikaten oder Flammenhydrolyse von Siliziumhalogeniden mit spezifischen Oberflächen von 5 - 1000, vorzugsweise 20 - 400 m²/g (BET-Oberfläche) und mit Primärteilchengrößen von 10 - 400 nm. Die Kieselsäuren können gegebenenfalls auch als Mischoxide mit anderen Metalloxiden wie Al-, Mg-, Ca-, Ba-, Zn-, Zr-, Ti-Oxiden vorliegen.
- synthetische Silikate, wie Aluminiumsilikat, Erdalkalisilikate wie Magnesium- oder Calciumsilikat, mit BET-Oberflächen von 20 - 400 m²/g und Primärteilchengröße von 10-400 nm,
- natürliche Silikate, wie Kaolin und andere natürliche vorkommende Kieselsäuren,
- Glasfasern auch in Formen von Matten und Strängen,
- Mikroglaskugeln.

Es können selbstverständlich zusätzliche Füllstoffe verwendet werden. Insbesondere sind hierfür nach dem Flammruß, Furnace- oder Gasruß-Verfahren hergestellt Ruße geeignet, welche BET-Oberflächen von 20 - 200 m²/g besitzen, wie SAF-, ISAF-, IISAF-, HAF-, FEF- oder GPF-Ruße.

Der Gesamtgehalt an Füllstoffen beträgt vorzugsweise 10 bis 200 phr, besonders bevorzugt 50 bis 160 phr und ganz besonders bevorzugt 60 bis 120 phr.

Eine besonders bevorzugte Ausführungsform stellt die Kombination von Kieselsäure, Ruß und erfindungsgemäßer Polysulfidmischung dar. Dabei kann das Verhältnis von Kieselsäure zu Ruß in beliebigen Grenzen variieren, wobei für die Anwendung in Reifen ein Kieselsäure : Ruß - Gewichtsverhältnis von 20 : 1 bis 1,5 : 1 bevorzugt ist.

In einer bevorzugten Ausführungsform enthalten die erfindungsgemäßen Kautschukmischungen auch einen oder mehrere Vernetzer. Hierfür sind insbesondere Schwefel-basierende oder peroxidische Vernetzer geeignet, wobei Schwefel-basierte Vernetzer besonders bevorzugt sind.

Als peroxidische Vernetzer werden vorzugsweise Bis(2,4-dichlorbenzyl)peroxid, Dibenzoylperoxid, Bis(4-chlorbenzoyl)peroxid, 1,1-Bis(t-butylperoxy)-3,3,5-trimethyl-cylohexan, tert-Butylperbenzoat, 2,2-Bis(t-butylperoxy)butan, 4,4-Di-tert-butylperoxynonyl-valerat, Dicumylperoxid, 2,5-Dimethyl-2,5-di(tert-butylperoxy)hexan, tert-Butylcumylperoxid, 1,3-Bis(t-butylperoxyisopropyl)benzol, Di-tert-butylperoxid und 2,5-Dimethyl-2,5-di(tert-butylperoxy)-3-hexin eingesetzt.

Es kann vorteilhaft sein, neben diesen peroxidischen Vernetzern noch weitere Zusätze zu verwenden, mit deren Hilfe die Vernetzungsausbeute erhöht werden kann: Hierfür sind beispielsweise Triallylisocyanurat, Triallylcyanurat, Trimethylolpropantri(meth)acrylat, Triallyltrimellitat, Ethylenglycoldi(meth)acrylat, Butandioldi(meth)acrylat, Trimetylolpropan-tri(meth)acrylat, Zinkdiacrylat, Zinkdimethacrylat, 1,2-Polybutadien oder N,N'-m-Phenylen-dimaleinimid geeignet.

Als Vernetzer kann Schwefel in elementarer löslicher oder unlöslicher Form oder in Form von Schwefelspendern eingesetzt werden. Als Schwefelspender kommen beispielsweise Dimorpholyldisulfid (DTDM), 2-Morpholinodithiobenzothiazol (MBSS), Caprolactamdisulfid, Dipentamethylenthiuramtetrasulfid (DPTT) und Tetramethylthiuramdisulfid (TMTD) in Frage.

Grundsätzlich kann die Vernetzung der erfindungsgemäßen Kautschukmischungen mit Schwefel oder Schwefelspendern allein erfolgen, oder zusammen mit Vulkanisationsbeschleunigern, wofür z.B. Dithiocarbamate, Thiurame, Thiazole, Sulfenamide, Xanthogenate, bi- oder polycyclische Amine, Guanidinderivate, Dithiophosphate, Caprolactame und Thioharnstoffderivate geeignet sind. Weiterhin sind auch Zinkdiamindiisocyanat, Hexamethylentetramin, 1,3-Bis(citraconimidomethyl)benzol sowie zyklische Disulfane geeignet. Bevorzugt enthalten die erfindungsgemäßen Kautschukmischungen Schwefel-basierende Vernetzer und Vulkanisationsbeschleuniger.

Besonders bevorzugt werden als Vernetzungsmittel Schwefel, Magnesiumoxid und/oder Zinkoxid eingesetzt, denen die bekannten Vulkanisationsbeschleuniger, wie Merkaptobenzothiazole, Thiazolsulfenamide, Thiurame, Thiocarbamate, Guanidine, Xanthogenate und Thiophosphate zugesetzt werden.

Die Vernetzungsmittel und Vulkanisationsbeschleuniger werden bevorzugt in Mengen von 0,1 bis 10 phr, besonders bevorzugt von 0,1 bis 5 phr, eingesetzt.

Die erfindungsgemäßen Kautschukmischungen können weitere Kautschukhilfsmittel enthalten, wie Reaktionsbeschleuniger, Alterungsschutzmittel, Wärmestabilisatoren, Lichtschutzmittel, Antioxidantien, insbesondere Ozonschutzmittel, Flammschutzmittel, Verarbeitungshilfsmittel, Schlagzähfestigkeitsverbesserer, Weichmacher, Tackifier, Treibmittel, Farbstoffe, Pigmente, Wachse, Streckmittel, organische Säuren, Verzögerer, Metalloxide und Aktivatoren, insbesondere Triethanolamin, Polyethylenglykol, Hexantriol und Reversionsschutzmittel.

Diese Kautschukhilfsmittel werden in üblichen Mengen eingesetzt, die sich u. a. nach dem Verwendungszweck der Vulkanisate richten. Übliche Mengen sind 0,1 bis 30 phr.

Als Alterungsschutzmittel werden bevorzugt alkylierte Phenole, styrolisiertes Phenol, sterisch gehinderte Phenole wie 2,6-Di-tert.-butylphenol, 2,6-Di-tert-butyl-p-kresol (BHT), 2,6-Di-tert.-butyl-4-ethylphenol, estergruppenhaltige sterisch gehinderte Phenole, thioetherhaltige sterisch gehinderte Phenole, 2,2'-Methylen-bis-(4-methyl-6-tert-butylphenol) (BPH) sowie sterisch gehinderte Thiobisphenole verwendet.

Falls eine Verfärbung des Kautschuks ohne Bedeutung ist, können auch aminische Alterungsschutzmittel z. B. Mischungen aus Diaryl-p-phenylendiaminen (DTPD), octyliertes Diphenylamin (ODPA), Phenyl-α-naphthylamin (PAN), Phenyl-β-naphthylamin (PBN), vorzugsweise solche auf Phenylendiaminbasis, z. B. N-Isopropyl-N'-phenyl-p-phenylendiamin, N-1,3-Dimethylbutyl-N'-Phenyl-p-phenylendiamin (6PPD), N-1,4-Dimethylpentyl-N'-phenyl-p-phenylendiamin (7PPD), N,N'-bis-(1,4-Dimethylpentyl)-p-phenylendiamin (77PD) eingesetzt werden.

Weitere Alterungsschutzmittel sind Phosphite wie Tris-(nonylphenyl)phosphit, polymerisiertes 2,2,4-Trimethyl-1,2-dihydrochinolin (TMQ), 2-Mercaptobenzimidazol (MBI), Methyl-2-Mercaptobenzimidazol (MMBI), Zinkmethylmercaptobenzimidazol (ZMMBI), welche meist in Kombination mit obigen phenolischen Alterungsschutzmitteln eingesetzt werden. TMQ, MBI und MMBI werden vor allem für NBR-Kautschuke verwendet, welche peroxidisch vulkanisiert werden.

Die Ozonbeständigkeit kann durch Antioxidantien wie beispielsweise N-1,3-Dimethylbutyl-N'-phenyl-p-phenylendiamin (6PPD), N-1,4-Dimethylpentyl-N'-phenyl-p-phenylendiamin (7PPD), N,N'-Bis- (1,4-dimethylpentyl)-p-phenylendiamin (77PD), Enolether oder cyclische Acetale. verbessert werden.

Verarbeitungshilfsmittel sollen zwischen den Kautschuk-Partikeln wirksam werden und Reibungskräften beim Mischen, Plastifizieren und Verformen entgegenwirken. Als Verarbeitungshilfsmittel können die erfindungsgemäßen Kautschukmischungen alle für die Verarbeitung von Kunststoffen üblichen Gleitmittel enthalten, wie beispielsweise Kohlenwasserstoffe, wie Öle, Paraffine und PE-Wachse, Fettalkohole mit 6 bis 20 C-Atomen, Ketone, Carbonsäuren, wie Fettsäuren und Montansäuren, oxidiertes PE-Wachs, Metallsalze von Carbonsäuren, Carbonsäureamide sowie Carbonsäureester, beispielsweise mit den Alkoholen Ethanol, Fettalkoholen, Glycerin, Ethandiol, Pentaerythrit und langkettigen Carbonsäuren als Säurekomponente.

Um die Entflammbarkeit zu vermindern und die Rauchentwicklung beim Verbrennen zu verringern, kann die erfindungsgemäße Kautschukmischungszusammensetzung auch Flammschutzmittel enthalten. Hierfür werden beispielsweise Antimontrioxid, Phosphorsäureester, Chlorparaffin, Aluminiumhydroxid, Borverbindungen, Zinkverbindungen, Molybdäntrioxid, Ferrocen, Calciumcarbonat oder Magnesiumcarbonat verwendet.

Vor der Vernetzung können dem Kautschukvulkanisat auch weitere Kunststoffe beigefügt werden, welche beispielsweise als polymere Verarbeitungshilfsmittel oder Schlagzähigkeitsverbesserer wirken. Diese Kunststoffe werden bevorzugt gewählt aus der Gruppe bestehend aus den Homo- und Copolymeren auf Basis von Ethylen, Propylen, Butadien, Styrol, Vinylacetat, Vinylchlorid, Glycidylacrylat, Glycidylmethacrylat, Acrylaten und Methacrylaten mit Alkoholkomponenten von verzweigten oder unverzweigten C₁- bis C₁₀-Alkoholen, wobei Polyacrylate mit gleichen oder verschiedenen Alkoholresten aus der Gruppe der C₄- bis C₈-Alkohole, insbesondere des Butanols, Hexanols, Octanols und 2-Ethylhexanols, Polymethylmethacrylat, Methylmethacrylat-Butylacrylat-Copolymere, Methylmethacrylat-Butylmethacrylat-Copolymere, Ethylen-Vinylacetat-Copolymere, chloriertes Polyethylen, Ethylen-Propylen-Copolymere, Ethylen-Propylen-Dien-Copolymere besonders bevorzugt sind.

In einer bevorzugten Ausführungsform enthält die erfindungsgemäße Kautschukmischung 0,1 bis 15 phr des Reversionsschutzmittels 1,6-Bis(N,N-dibenzylthiocarbamoyldithio)-hexan (CAS-Nr.: 151900-44-6), wodurch eine Verringerung von tan δ (60 °C), d.h. des Rollwiderstands ermöglicht wird, die Abriebwerte verbessert und die Anvulkanisationszeit und Ausvulkanisationszeit verkürzt werden können.

Bevorzugt zeichnen sich die erfindungsgemäßen Kautschukmischungen dadurch aus, dass ein daraus bei 170°C/t95 geheiztes Vulkanisat einen Verlustfaktor tan δ bei 60°C von < 0,16, besonders bevorzugt < 0,12, insbesondere < 0,11 und gleichzeitig eine Härte Shore A bei 23°C von > 66 aufweist. In Kombination damit können durch die erfindungsgemäßen Kautschukmischungen auch eine Ausvulkanisationszeit von kleiner 2000 Sekunden erreicht werden.

Ein weiterer Gegenstand der vorliegenden Erfindung ist ein Verfahren zur Herstellung von Kautschukmischungen, durch Mischen von mindestens einem Kautschuk mit mindestens einem kieselsäurebasierenden Füllstoff, einem schwefelhaltigen Alkoxysilan und mindestens einer erfindungsgemäßen Polysulfidmischung. Vorzugsweise werden dabei 10 bis 150 phr, besonders bevorzugt 30 bis 120 phr und ganz besonders bevorzugt 50 bis 100 phr Füllstoff, 0,1 bis 15 phr, besonders bevorzugt 0,3 bis 7 phr, ganz besonders bevorzugt 0,5 bis 3 phr und am meisten bevorzugt 0,7 bis 1,5 phr erfindungsgemäße Polysulfidmischung sowie 2 bis 20 phr, besonders bevorzugt 3-11 phr und ganz besonders bevorzugt 5 bis 8 phr des schwefelhaltigen Alkoxysilans eingesetzt. Weiterhin können im Mischverfahren die oben genannten zusätzlichen Füllstoffe, Vernetzer, Vulkanisationsbeschleuniger und Kautschukhilfsmittel, vorzugsweise in den oben angegebenen Mengen zugesetzt werden.

Bei dem mehrstufigen Mischverfahren erfolgt die Zugabe der erfindungsgemäßen Polysulfidmischung bevorzugt im ersten Teil des Mischprozesses und die Zugabe von einem oder mehreren Vernetzern, insbesondere Schwefel, und ggf. Vulkanisationsbeschleuniger in einer späteren Mischstufe. Die Temperatur der Kautschukmasse beträgt dabei vorzugsweise 100 bis 200 °C, besonders bevorzugt 120°C bis 170 °C. Die Scherraten bei der Mischung betragen 1 bis 1000 sec⁻¹, bevorzugt 1 bis 100 sec⁻¹. In einer bevorzugten Ausführungsform wird die Kautschukmischung nach der ersten Mischstufe abgekühlt und der Vernetzer und ggf. Vernetzungsbeschleuniger und/oder Zusätze mit deren Hilfe die Vernetzungsausbeute erhöht wird, in einer späteren Mischstufe bei < 140°C, vorzugsweise < 100°C zugegeben. Die Zugabe der erfindungsgemäßen Polysulfidmischung in einer späteren Mischstufe und bei tieferen Temperaturen wie 40 bis 100°C ist ebenfalls möglich z.B. zusammen mit Schwefel und Vernetzungsbeschleuniger.

Die Abmischungen des Kautschuks mit dem Füllstoff und der erfindungsgemäßen Polysulfidmischung kann in üblichen Mischaggregaten, wie Walzen, Innenmischer und Mischextruder, durchgeführt werden.

Die optionale Zugabe von 1,6-Bis(N,N-dibenzylthiocarbamoyldithio)hexan findet vorzugsweise in der ersten Stufe des mehrstufigen Mischverfahrens statt.

Ein weiterer Gegenstand der vorliegenden Erfindung ist ein Verfahren zur Vulkanisation der erfindungsgemäßen Kautschukmischungen, welche bevorzugt bei Massetemperaturen von 100 bis 200 °C, besonders bevorzugt bei 130 bis 180 °C durchgeführt wird. In einer bevorzugten Ausführungsform geschieht die Vulkanisation bei einem Druck von 10 bis 200 bar.

Die vorliegende Erfindung umfasst auch Kautschukvulkanisate erhältlich durch Vulkanisation der erfindungsgemäßen Kautschukmischungen, sowie Kautschukprodukte enthaltend diese Vulkanisate, insbesondere Reifen, da entsprechende Reifen den Vorteil einer hohen Härte gepaart mit einem guten Rollwiderstand und niedrigem Abrieb aufweisen.

Die Ausrüstung von Fahrzeugen insbesondere Kraftfahrzeugen mit Reifen, welche die erfindungsgemäßen Vulkanisate enthalten, führt zu einem geringeren Energieaufwand beim Betrieb dieser Fahrzeuge, wodurch bei Kraftfahrzeugen mit Verbrennungsmotoren ein geringerer Kraftstoffverbrauch, bei Fahrzeugen mit Elektroantrieb eine größere Reichweite und bei muskelbetriebenen Fahrzeugen eine geringere Anstrengung und/oder eine höhere Geschwindigkeit ermöglicht wird. Daher umfasst die vorliegende Erfindung auch Fahrzeuge enthaltend Kautschukprodukte, welche die erfindungsgemäßen Vulkanisate beinhalten.

Die erfindungsgemäßen Kautschukvulkanisate eignen sich zur Herstellung von Formkörpern mit verbesserten Eigenschaften, z.B. für die Herstellung von Kabelmäntel, Schläuchen, Treibriemen, Förderbändern, Walzenbelägen, Reifen, Schuhsohlen, Dichtungsringen und Dämpfungselementen.

Das erfindungsgemäße Kautschukvulkanisat kann zudem zur Herstellung von Schaumstoffen verwendet werden. Dazu werden ihr chemisch oder physikalisch wirkende Treibmittel zugefügt. Als chemisch wirkende Treibmittel kommen alle für diesen Zweck bekannten Substanzen in Betracht, wie beispielsweise Azodicarbonamid, p-Toluolsulfonylhydrazid, 4,4'-Oxybis(benzolsulfohydrazid), p-Toluolsulfonylsemicarbazid, 5-Phenyltetrazol, N,N'-Dinitroso-pentamethylentetramin, Zinkcarbonat oder Natriumhydrogencarbonat sowie diese Substanzen enthaltende Mischungen. Als physikalisch wirkende Treibmittel sind beispielsweise Kohlendioxid oder Halogenkohlenwasserstoffe geeignet.

Ein weiterer Gegenstand der vorliegenden Erfindung ist die Verwendung der erfindungsgemäßen Polysulfidmischung zur Herstellung von Kautschukmischungen, und deren Vulkanisaten insbesondere zur Herstellung von Kautschukmischungen enthaltend zumindest jeweils einen Kautschuk, ein schwefelhaltiges Alkoxysilan und einen kieselsäurebasierenden Füllstoff.

Überraschenderweise wurde gefunden, dass Additivzusammensetzungen für Kautschuke enthaltend zumindest ein schwelfelhaltiges Alkoxysilan, insbesondere Bis-(triethoxysilyl-propyl)tetrasulfan, Bis-(triethoxysilylpropyl)disulfan, 3-(Triethoxysilyl)-1-propanthiol, polyetherfunktionalisiertes Mercaptosilan oder thioesterfunktionalisiertes Alkoxysilan und die erfindungsgemäße Polysulfidmischung trotz der reaktiven Gruppen eine ausreichende Verträglichkeit der Komponenten aufweisen, wodurch eine homogene Einbringung in Kautschukmischungen und eine exakte Dosierung im gewünschten Verhältnis ermöglicht wird. Daher umfasst die vorliegende Erfindung auch solche Additivzusammensetzungen für Kautschuke, sowie die Verwendung der erfindungsgemäßen Polysulfidmischungen zur Herstellung solcher Additivzusammensetzungen. In diesen Additivzusammensetzungen beträgt das Gewichtsverhältnis von Alkoxysilan, insbesondere von Bis-(triethoxysilyl-propyl)tetrasulfan und/oder von Bis-(triethoxysilylpropyl)disulfan zu den erfindungsgemäßen Polysulfidmischungen vorzugsweise 1,5:1 bis 20:1, besonders bevorzugt 3:1 bis 15:1 und ganz besonders bevorzugt 5:1 bis 10:1.

Zudem umfasst die vorliegende Erfindung ein Verfahren zur Herstellung von Additivzusammensetzungen für Kautschuke, dadurch gekennzeichnet, dass man schwelfelhaltige Alkoxysilane mit den erfindungsgemäßen Polysulfidmischungen mischt.

Weiterhin umfasst die vorliegende Erfindung ein Verfahren zur Verringerung des Rollwiderstands von Reifen, bei welchem man einer nicht oder teilvernetzten Kautschukmischung welche als Ausgangsmaterial für zumindest Teile des Reifen dient, eine erfindungsgemäße Polysulfidmischung beimengt und die Mischung anschließend vulkanisiert.

### Bestimmung der Eigenschaften von Kautschukmischung bzw. Vulkanisaten:

### Mooney-Viskositätsmessung:_

Die Viskosität lässt sich aus der Kraft, die Kautschuke (und Kautschukmischungen) ihrer Verarbeitung entgegensetzen, direkt bestimmen. Beim Scherscheibenviskosimeter nach Mooney wird eine geriffelte Scheibe oben und unten mit Probensubstanz umschlossen und in einer beheizbaren Kammer mit etwa zwei Umdrehungen in der Minute bewegt. Die hierzu erforderliche Kraft wird als Drehmoment gemessen und entspricht der jeweiligen Viskosität. Die Probe wird in der Regel eine Minute lang auf 100°C vorgewärmt; die Messung dauert weitere 4 Minuten, wobei die Temperatur konstant gehalten wird. Die Viskosität wird zusammen mit den jeweiligen Prüfbedingungen angegeben, beispielsweise ML (1+4) 100°C (Mooney viscosity, large rotor, Vorwärmzeit und Prüfzeit in Minuten, Prüftemperatur).

### Scorch-Verhalten (Anvulkanisationszeit t 5):

Des Weiteren kann mit der gleichen Prüfung wie oben beschrieben auch das Scorch-Verhalten einer Mischung gemessen werden. Die gewählte Temperatur betrug 130°C. Der Rotor läuft solang, bis der Drehmomentswert nach Durchlaufen eines Minimums auf 5 Mooney-Einheiten relativ zum Minimumswert angestiegen ist (t5). Je größer der Wert ist (Einheit Sekunden), umso langsamer findet die Anvulkanisation statt. In der Praxis ist meist eine Anvulkanisationszeit von mehr als 300 Sekunden vorteilhaft.

### Rheometer (Vulkameter) Ausvulkanisationszeit 170°C/t95:

Der Vulkanisationsverlauf am MDR (moving die rheometer) und dessen analytischen Daten werden an einem Monsanto-Rheometer MDR 2000 nach ASTM D5289-95 gemessen. Als Ausvulkanisationszeit, wird die Zeit bestimmt, bei der 95% des Kautschuks vernetzt ist. Die gewählte Temperatur betrug 170°C.

### Bestimmung der Härte:

Zur Bestimmung der Härte der erfindungsgemäßen Kautschukmischung wurden 6 mm starke Walzfelle aus der Kautschukmischung gemäß Rezepturen der Tabelle 1 hergestellt. Aus den Walzfellen wurden Prüfkörper mit 35 mm Durchmesser geschnitten, deren Shore-Härte A mittels eines digitalen Shore-Härte-Testers (Zwick GmbH & Co. KG, Ulm) bestimmt wurden. Die Härte eines Kautschukvulkanisates gibt einen ersten Hinweis über dessen Steifigkeit wieder.

### Zugversuch:

Der Zugversuch dient direkt zur Ermittlung der Belastungsgrenzen eines Elastomers und erfolgt nach DIN 53504. Die Längenausdehnung beim Bruch wird auf die Ausgangslänge bezogen und entspricht der Bruchdehnung. Weiterhin wird auch die Kraft beim Erreichen bestimmter Dehnungsstufen, meist 50, 100, 200 und 300% bestimmt und als Spannungswert ausgedrückt (Zugfestigkeit bei der angegebenen Dehnung von 300% oder Modul 300).

### Dyn. Dämpfung:

Dynamische Prüfverfahren werden zur Charakterisierung des Verformungsverhaltens von Elastomeren unter periodisch veränderten Belastungen verwendet. Eine von außen angebrachte Spannung verändert die Konformation der Polymerkette. Der Verlustfaktor tan δ wird dabei indirekt über das Verhältnis zwischen Verlustmodul G" und Speichermodul G' bestimmt. Der Verlustfaktor tan δ bei 60°C geht mit dem Rollwiderstand einher und sollte möglichst niedrig sein.

### Abrieb:

Der Abrieb gibt einen Hinweis auf die Abnutzung und damit auf die Lebensdauer eines Produktes. Der Abrieb wurde nach DIN 53516 bestimmt. Aus ökonomischen und ökologischen Gründen ist ein niedriger Wert anzustreben.

### Beispiel 1:

- Apparatur:: 2000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr
- Vorlage:: 99,1 g (0,25 mol) Dinatriumhexamethylen-1,6-bisthiosulfatdihydrat Duralink HTS von Flexsys (98,48%)
600 ml vollentsalztes Wasser
41,1 g (0,5 mol) Formaldehydlösung, ca. 36,5%ig
42 g (0,5 mol) Natriumhydrogencarbonat
- Zulauf:: 78,7 g (0,5 mol) 2-Mercaptobenzoesäure (98%), unter Stickstoff gelöst in 500 ml Wasser bei pH 8 durch NaOH-Zugabe
- Hilfsmittel:: 37%ige HCl

In der mit Stickstoff gespülten Apparatur wurde Duralink HTS und Wasser vorgelegt. Unter Rühren wurden erst Natriumhydrogencarbonat und danach Formaldehyd zugegeben. Anschließend wurde bei einer Temperatur von 20 - 25°C die 2-Mercaptobenzoesäure-Lösung unter Stickstoffüberleitung innerhalb von ca. einer 1 h zugetropft. Nach beendeter Dosierung wurde 22h nachgerührt und dann unter Stickstoffüberleitung bei 20 - 25 °C der pH-Wert mit 37%iger Salzsäure auf pH 2 eingestellt. Während der pH-Einstellung schäumte der Ansatz sehr stark. Es wurde eine Stunde nachgerührt und der Feststoff anschließend mittels D4-Fritte abgesaugt. Anschließend wurde das Produkt mit Portionen von je 300 ml Wasser gewaschen, bis die Leitfähigkeit des Waschwassers < 0,3 mS/cm betrug, und dann bei 25°C im Vakuumtrockenschränk getrocknet.

Ausbeute: 113,3 g (99,7%) einer Mischung aus Polysulfiden der Formel (I) mit m = 0, 1 und 2

Elementaranalyse: C: 52,3% H: 5,2% O: 14,3% S: 28,0% Cl: 1200ppm

Das Produkt wurde durch RP-HPLC und Time-of-Flight Mass Spectrometry (TOF MS) analysiert. Die Prozentangaben zu den Verbindungen der Formel (I) ergaben sich aus den Flächenprozentanteilen der HPLC-Messung mit UV-Detektor. Erhalten wurden folgende Verbindungen der Formel (I) mit K₁⁺ und K₂⁺ = H⁺:
25% Verbindung mit m = 0; 54% Verbindung mit m =1; 21% Verbindung mit m = 2.

### Beispiel 2

- Apparatur:: 4000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr, Dulcometer
- Vorlage:: 198,0 g (0,5 mol) Dinatriumhexamethylen-1,6-bisthiosulfatdihydrat Duralink HTS von Flexsys (98,59%)
1200 ml vollentsalztes Wasser
82,3 g (1,0 mol) Formaldehydlösung, ca. 36,5%ig
- Zulauf:: 157,4 g (1,0 mol) 2-Mercaptobenzoesäure (98%), unter Stickstoff gelöst in 1000 ml Wasser durch 60g NaOH-Zugabe
- Hilfsmittel:: 292 g (0,4 mol) 5%ige HCl (Dosierung über Dulcometer)
108,4 g (1,1 mol) 37%ige HCl

In der mit Stickstoff gespülten Apparatur wurden Duralink HTS und Wasser vorgelegt. Unter Rühren wurde Formaldehyd zugegeben. Anschließend wurde bei einer Temperatur von 5°C 2-Mercaptobenzoesäure-Lösung unter Stickstoffüberleitung innerhalb von ca. 90min zugetropft. Der Reaktionsansatz wurde durch Zutropfen einer 5%igen HCl während der Zugabe der 2-Mercaptobenzoesäure-Lösung auf pH 9,5 - 10,5 gehalten. Nach beendeter Zugabe wurde 1 h bei 5°C nachgerührt und dann unter Stickstoffüberleitung bei 5 °C die 37%ige Salzsäure in 1h zugetropft. Es wurde eine Stunde nachgerührt und der Feststoff anschließend mittels D4-Fritte abgesaugt. Danach wurde das Produkt mit Portionen von jeweils 600 ml Wasser solange gewaschen, bis die Leitfähigkeit des Waschwassers < 0,3 mS/cm betrug, und dann bei 50°C im Vakuumtrockenschrank getrocknet.

Ausbeute: 233 g (102,5%) einer Mischung aus Polysulfiden der Formel (I) mit m = 0, 1 und 2, mit K₁⁺ und K₂⁺ = H⁺

Elementaranalyse: C: 52,4% H: 5,1% O: 14,3% S: 27,7% Cl: 130ppm

Die Prozentangaben zu den Verbindungen der Formel (I) ergaben sich aus den Flächenprozentanteilen der HPLC-Messung mit UV-Detektor

3% Verbindung mit m = 0; 96% Verbindung mit m = 1; 1% Verbindung mit m = 2.

### Beispiel 3

- Apparatur:: 2000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr, Dulcometer
- Vorlage:: 99,0 g (0,25 mol) Duralink HTS von Flexsys (98,59%)
600 ml vollentsalztes Wasser
41,1 g (0,5 mol) Formaldehydlösung, ca. 36,5%ig
- Zulauf:: 78,7 g (0,5 mol) 2-Mercaptobenzoesäure (98%), unter Stickstoff gelöst in 500 ml Wasser durch 30g NaOH Zugabe
- Hilfsmittel:: 98,1 g (0,1 mol) 10%ige H₂SO₄ (Dosierung über Dulcometer)
67,4 g (0,275 mol) 40%ige H₂SO₄

In der mit Stickstoff gespülten Apparatur wurden Duralink HTS und Wasser vorgelegt. Unter Rühren wurde Formaldehyd zugegeben. Anschließend wurden bei einer Temperatur von 5°C 2-Mercaptobenzoesäure-Lösung unter Stickstoffüberleitung innerhalb von ca. 90min zugetropft. Der Reaktionsansatz wurde durch Zutropfen einer 10%igen Schwefelsäure während der Zugabe der 2-Mercaptobenzoesäure-Lösung auf pH 9,5 - 10,5 gehalten. Nach beendeter Zugabe wurde 1 h bei 5°C nachgerührt und anschließend unter Stickstoffüberleitung bei 5 °C die 40%ige Schwefelsäure in 1 h zugetropft. Es wurde eine Stunde nachgerührt und der Feststoff anschließend mittels D4-Fritte abgesaugt. Danach wurde das Produkt mit Portionen von jeweils 300 ml Wasser so lange gewaschen, bis die Leitfähigkeit des Waschwassers < 0,3 mS/cm betrug, und dann bei 50°C im Vakuumtrockenschrank getrocknet.

Ausbeute: 116,7 g (102,7%) einer Mischung aus Polysulfiden der Formel (I) mit m = 0, 1 und 2, mit K₁⁺ und K₂⁺ = H⁺

Elementaranalyse:
C: 52,5% H: 5,0% O: 14,5% S: 28,2% Cl: < 10ppm

Die Prozentangaben zu den Verbindungen der Formel (I) ergaben sich aus den Flächenprozentanteilen der HPLC-Messung mit UV-Detektor.

3% Verbindung mit m = 0; 96% Verbindung m = 1; 1 % Verbindung mit m = 2.

HPLC-Gerät: Agilent 1100 Series mit Degaser, binärer Pumpe, Säulenofen, variablem Wellenlängendetektor und Autosampler
- Stationäre Phase:: Inertsil ODS-3, Teilchendurchmesser 3 µm
- Säulenlänge:: 150 mm
- Säuleninnendurchmesser:: 2,1 mm
- Mobile Phase A:: 100% Wasser + 25 mmol/L Ammoniumacetat
- B:: 95% Methanol : 5% Wasser + 25 mmol/L Ammoniumacetat

### Elutionsprogram

| Zeit (min) | Eluent A (Vol%) | Eluent B (Vol%) |
|---|---|---|
| 0 | 80 | 20 |
| 5 | 80 | 20 |
| 30 | 1 | 99 |
| 60 | 1 | 99 |
| 62 | 80 | 20 |

- Säulentemperatur:: 40°C
- Fließgeschwindigkeit:: 0,3 ml/min
- Laufzeit:: 72 min
- Injektionsvolumen:: 5 µl
- Wellenlänge des UV-Detektors:: 225 nm

Eine ca. 50 mg Probe zu analysierendes Produkt wurde in einem 50 ml Messkolben eingewogen, mit 2 ml Dimethylsulfoxid versetzt, mit Tetrahydrofuran bis zur Eichmarke aufgefüllt, homogenisiert und direkt im Anschluss chromatographiert.

### Beispiel 4

- Apparatur:: 2000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr, Dulcometer
- Vorlage:: 99,0 g (0,25 mol) Duralink HTS von Flexsys (98,59%)
600 ml vollentsalztes Wasser
41,1 g (0,5 mol) Formaldehydlösung, ca. 36,5%ig
- Zulauf 1:: 78,7 g (0,5 mol) 2-Mercaptobenzoesäure (98%), unter Stickstoff gelöst in 500 ml Wasser durch 30g NaOH-Zugabe
- Hilfsmittel:: 146 g (0,2 mol) 5%ige HCl (Dosierung über Dulcometer)
- Zulauf 2:: 44,8 g (0,25 mol) ZnSO₄ x H₂O in 375 ml Wasser

In der mit Stickstoff gespülten Apparatur wurden Duralink HTS und Wasser vorgelegt. Unter Rühren wurde Formaldehyd zugegeben. Anschließend wurde bei einer Temperatur von 5°C 2-Mercaptobenzoesäure-Lösung unter Stickstoffüberleitung innerhalb von ca. 90min zugetropft. Der Reaktionsansatz wurde durch Zutropfen einer 5%igen Salzsäure während der Zugabe der 2-Mercaptobenzoesäure-Lösung auf pH 9,5 - 10,5 gehalten. Nach beendeter Zugabe wurde 1h bei 5°C nachgerührt und dann unter Stickstoffüberleitung bei 5 °C die Zinksulfat-Lösung in 1h zugetropft. Es wurde eine Stunde nachgerührt und der Feststoff anschließend mittels D4-Fritte abgesaugt. Danach wurde das Produkt mit Portionen von jeweils 300 ml Wasser so lange gewaschen, bis die Leitfähigkeit des Waschwassers < 0,3 mS/cm betrug, und dann bei 50°C im Vakuumtrockenschrank getrocknet.

Ausbeute: 124,8 g (96,4%) einer Polysulfidmischung der Formel (I) mit K₁⁺ und K₂⁺ = ½Zn²⁺ :
Elementaranalyse:
   C: 45,7% H: 4,2% O: 13,4% S: 24,5% Zn: 12% Cl: 120ppm

Die Prozentangaben zu den Verbindungen der Formel (I) ergaben sich aus den Flächenprozentanteilen der HPLC-Messung mit UV-Detektor.

4% Verbindung mit m = 0; 91% Verbindung m = 1; 5% Verbindung mit m = 2.

### Herstellung von Kautschukmischungen und Kautschukvulkanisaten

Die in Tabelle 1 aufgeführten Kautschuk-Rezepturen wurden jeweils gemäß nachfolgend beschriebenen, mehrstufigen Verfahren gemischt.
1. Mischstufe:
   ▪ BUNA^{®} CB 24 und BUNA^{®} VSL 5025-2 wurde in einem Innenmischer vorgelegt und ca. 30 Sekunden gemischt
   ▪ Zugabe zwei Drittel VULKASIL^{®} S, zwei Drittel SI^{®} 69, zwei Drittel der Gesamtmenge an erfindungsgemäßer Polysulfidmischung, mischen für ca. 60 Sekunden
   ▪ Zugabe ein Drittel VULKASIL^{®} S, ein Drittel SI^{®} 69, ein Drittel der Gesamtmenge an erfindungsgemäßer Polysulfidmischung sowie TUDALEN 1849-1, mischen ca. 60 Sekunden
   Zugabe von CORAX^{®} N 339, EDENOR^{®} C 18 98-100, VULKANOX^{®} 4020/LG, VULKANOX^{®} HS/LG, ZINKWEISS ROTSIEGEL sowie ANTILUX^{®} 654, mischen für ca. 60 Sekunden. Die Mischung erfolgte bei einer Temperatur von 150°C.
2. Mischstufe:
   Nach Abschluss der ersten Mischstufe wurde das Mischstück von einem nachgeschalteten Walzwerk aufgenommen und zu einer Platte ausgeformt und für 24 Stunden bei Raumtemperatur gelagert. Die Verarbeitungstemperaturen lagen hierbei unter 60°C.
3. Mischstufe:
   In der dritten Mischstufe erfolgte ein Nachzwicken bei 150°C in einem Kneter.
4. Mischstufe:
   Zugabe der Zusatzstoffe MAHLSCHWEFEL 90/95 CHANCEL, VULKACIT^{®} CZ/C, VULKACIT^{®} D/C auf einer Walze bei Temperaturen unter 80°C.

Die Kautschukmischungen wurden anschließend bei 170°C ausvulkanisiert. Die Eigenschaften der hergestellten Kautschukzubereitungen und deren Vulkanisate sind in Tabelle 2 angegeben.

**Tabelle 1: Kautschuk-Rezeptur**

| | **Referenz** | **Kautschuk -rezeptur 1** | **Kautschuk -rezeptur 2** | **Kautschuk -rezeptur 3** | **Kautschuk -rezeptur 4** |
|---|---|---|---|---|---|
| BUNA CB 24 | 30 | 30 | 30 | 30 | 30 |
| BUNA VSL 5025-2 | 96 | 96 | 96 | 96 | 96 |
| CORAX N 339 | 6,4 | 6,4 | 6,4 | 6,4 | 6,4 |
| VULKASIL S | 80 | 80 | 80 | 80 | 80 |
| TUDALEN 1849-1 | 8 | 8 | 8 | 8 | 8 |
| EDENOR C 18 98-100 | 1 | 1 | 1 | 1 | 1 |
| VULKANOX 4020/LG | 1 | 1 | 1 | 1 | 1 |
| VULKANOX HS/LG | 1 | 1 | 1 | 1 | 1 |
| ZINKWEISS ROTSIEGEL | 2,5 | 2,5 | 2,5 | 2,5 | 2,5 |
| ANTILUX 654 | 1,5 | 1,5 | 1,5 | 1,5 | 1,5 |
| SI 69 | 6,4 | 6,4 | 6,4 | 6,4 | 6,4 |
| VULKACIT D/C | 2 | 2 | 2 | 2 | 2 |
| VULKACIT CZ/C | 1,5 | 1,5 | 1,5 | 1,5 | 1,5 |
| MAHLSCHWEFEL 90/95 CHANCEL | 1,5 | 1,5 | 1,5 | 1,5 | 1,5 |
| Verbindung Beispiel 1 | | 1 | | | |
| Verbindung Beispiel 2 | | | 1 | | |
| Verbindung Beispiel 3 | | | | 1 | |
| Verbindung Beispiel 4 | | | | | 1 |

Mengenangaben in phr (Gewichtsteile pro 100 Teile Kautschuk)

| **Handelsname** | **Erläuterung** | **Hersteller/Vertrieb** |
|---|---|---|
| BUNA CB 24 | BR | Lanxess Deutschland GmbH |
| BUNA VSL 5025-2 | SBR | Lanxess Deutschland GmbH |
| CORAX N 339 | Ruß | Degussa-Evonik GmbH |
| VULKASIL S | Kieselsäure | Lanxess Deutschland GmbH |
| TUDALEN 1849-1 | Mineralöl | Hansen&Rosenthal KG |
| EDENOR C 18 98-100 | Stearinsäure | Cognis Deutschland GmbH |
| VULKANOX 4020/LG | N-1,3-Dimethylbutyl-N-phenyl-p-phenylendiamin | Lanxess Deutschland GmbH |
| VULKANOX HS/LG | 2,2,4-Trimethyl-1,2-dihydrochinolin polymerisiert | Lanxess Deutschland Gmbh |
| ZINKWEISS ROTSIEGEL | Zinkoxid | Grillo Zinkoxid GmbH |
| ANTILUX 654 | Lichtschutzwachs | RheinChemie Rheinau GmbH |
| SI 69 | Bis(triethoxysilylpropyl)tetrasulfid | Evonik Industries |
| VULKACIT D/C | 1,3-Diphenylguanidin | Lanxess Deutschland GmbH |
| VULKACIT CZ/C | N-cyclohexyl-2-benzothiazol-sulfenamid | Lanxess Deutschland GmbH |
| MAHLSCHWEFEL 90/95 CHANCEL | Schwefel | Solvay Deutschland GmbH |

**Tabelle 2: Zusammenstellung der Ergebnisse**

| **Parameter** | **Einheit** | **DIN** | **Referenz** | **Kautschuk -rezeptur 1** | **Kautschuk -rezeptur 2** | **Kautschuk -rezeptur 4** |
|---|---|---|---|---|---|---|
| Mooney Viskosität (ML 1+4) | [ME] | 53523 | 95 | 89 | 86 | 87 |
| Mooney Anvulkanisation bei 130°C (t5) | sec | ASTM D 5289-95 | 1253 | 1495 | 1078 | 991 |
| Ausvulkanisation bei 170°C / t95 | sec | 53529 | 1417 | 1588 | 1574 | 1523 |
| Shore A Härte bei 23°C | [Shore A] | 53505 | 66 | 72 | 67 | 68 |
| Modul 300 | MPa | 53504 | 15 | 15 | 15 | 15 |
| Bruchdehnung | % | 53504 | 349 | 338 | 322 | 370 |
| Zugfestigkeit | MPa | 53504 | 19 | 18 | 17 | 20 |
| Abrieb | mm³ | 53516 | 74 | 76 | 72 | 73 |
| Rollwiderstand (tan δ (60 °C)) | - | | 0,133 | 0,145 | 0,097 | 0,100 |

Die getesteten Vulkanisate zeigen im Vergleich zur Referenz erhöhte Härtewerte und verbesserte Mooney-Viskosität. Kautschukrezepturen 2 und 4 zeigen exzellenten Rollwiderstand und verbesserten Abrieb.

## Patentansprüche

1. Polysulfidmischungen enthaltend zwei oder mehr Verbindungen der Formel (I), 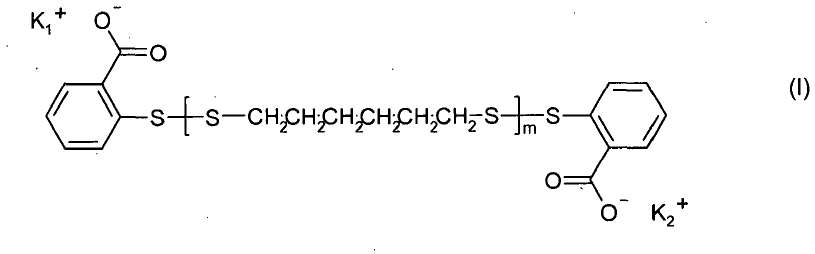 wobei K₁⁺ und K₂⁺ identisch oder verschieden, vorzugsweise identisch sind und unabhängig voneinander für ein beliebiges einwertiges Kation oder für den n-ten Teil eines beliebigen n-wertigen Kations stehen, und
m für 0, 1 und/oder 2 steht,
**dadurch gekennzeichnet, dass** der gesamte Anteil an Verbindung(en) mit m = 1 wenigstens 80%, bevorzugt wenigstens 85%, besonders bevorzugt wenigstens 90%, ganz besonders bevorzugt 93 - 99% beträgt, bezogen auf die Gesamtmenge an Polysulfidverbindungen der Formel (I) mit m = 0, 1 und 2.

2. Polysulfidmischung gemäß Anspruch 1, **dadurch gekennzeichnet, dass** K₁⁺ und K₂⁺ identisch oder verschieden, vorzugsweise identisch sind und unabhängig voneinander für für H⁺, ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺, besonders bevorzugt für H⁺ oder für ½ Zn²⁺ stehen.

3. Polysulfidmischung gemäß Anspruch 1 oder 2, **dadurch gekennzeichnet, dass** sie einen Gesamtchlorgehalt < 1%, bevorzugt < 0,1%, besonders bevorzugt < 200ppm, ganz besonders bevorzugt < 50ppm, am meisten bevorzugt < 10ppm hat.

4. Verfahren zur Herstellung einer Polysulfidmischung gemäß einem der Ansprüche 1 bis 3, **dadurch gekennzeichnet, dass** man eine oder mehrere Verbindungen der Formel (III) 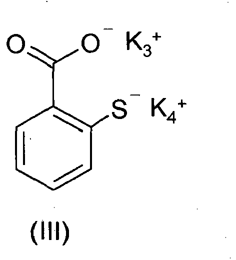 wobei Kationen K₃⁺ und K₄⁺ unabhängig voneinander für beliebige einwertige Kationen oder für den n-ten Teil eines beliebigen n-wertigen Kations, vorzugsweise für H⁺, für ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺, besonders bevorzugt für H⁺, Na⁺, K⁺ oder für ½ Zn²⁺ stehen,
mit ein oder mehreren Verbindungen der Formel (IV) in Kontakt bringt, 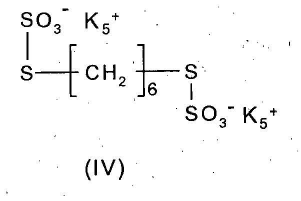 wobei die beiden Kationen K₅⁺ dabei gleich oder verschieden, vorzugsweise gleich sind und unabhängig voneinander für beliebige einwertige Kationen oder für den n-ten Teil eines beliebigen n-wertigen Kations, vorzugsweise für ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺, besonders bevorzugt für Na⁺ stehen,
**dadurch gekennzeichnet, dass** das Kontaktieren bei Temperaturen im Bereich von -5°C bis 19°C, bevorzugt von 0°C bis 15°C, besonders bevorzugt von 0°C bis 10°C erfolgt.

5. Verfahren gemäß Anspruch 4, **dadurch gekennzeichnet, dass** Verbindung(en) der Formel (III) mit K₃⁺ und/oder K₄⁺ ≠ H⁺ zugegeben werden und der pH der Reaktionsmischung im Bereich von pH 7 - 13, bevorzugt 8-12, insbesondere 9 - 11 gehalten wird, vorzugsweise durch Zugabe einer Brönsted-Säure.

6. Verfahren gemäß Anspruch 4 oder 5, **dadurch gekennzeichnet, dass** nach Kontaktieren der Verbindung der Formel (III) mit mindestens einer Verbindung der Formel (IV) die Polysulfidmischung durch Zugabe zumindest einer Säure, insbesondere Salzsäure oder Schwefelsäure, und/oder Inkontaktbringen mit mindestens einem Salz der Kationen K₁⁺ und/oder K₂⁺, wobei K₁⁺ und/oder K₂⁺ für ein anderes Kation als H⁺ stehen, ausgefällt wird.

7. Verfahren gemäß einem der Ansprüche 4 bis 6, **dadurch gekennzeichnet, dass** nach Kontaktieren der Verbindung der Formel (III) mit mindestens einer Verbindung der Formel (IV) die Polysulfidmischung durch Inkontaktbringen mit mindestens einem Salz der Kationen K₁⁺ und/oder K₂⁺, wobei K₁⁺ und/oder K₂⁺ für ein anderes Kation als H⁺ stehen, vorzugsweise einem Zinksalz, insbesondere Zinksulfat, ausgefällt wird.

8. Polysulfidverbindungen der Formel (I) gemäß Anspruch 1, wobei K₁⁺ und K₂⁺ identisch oder verschieden, vorzugsweise identisch sind und unabhängig voneinander für ein beliebiges einwertiges Kation oder für den n-ten Teil eines beliebigen n-wertigen Kations, vorzugsweise für H⁺, ein Alkalikation, insbesondere Li⁺, Na⁺, K⁺, ½ Erdalkalikation, insbesondere ½ Mg²⁺, ½ Ca²⁺, für 1/3 Al³⁺, für den n-ten Teil eines n-wertigen Seltenerdmetallkations, oder für ½ Zn²⁺, besonders bevorzugt für ½ Zn²⁺ stehen,
wobei K₁⁺ und/oder K₂⁺, vorzugsweise K₁⁺ und K₂⁺, für ein anderes Kation als H⁺ stehen und
m für 1 oder 2, vorzugsweise für 1 steht.

9. Kautschukmischung enthaltend jeweils zumindest
- einen Kautschuk
- eine Polysulfidmischung gemäß einem der Ansprüche 1 bis 3 oder Polysulfidverbindungen gemäß Anspruch 8.

10. Kautschukmischung gemäß Anspruch 9 enthaltend zusätzlich mindestens
- ein schwefelhaltiges Alkoxysilan, und
- einen kieselsäurebasierenden Füllstoff.

11. Kautschukmischung gemäß Anspruch 9 oder 10, **dadurch gekennzeichnet, dass** sie zusätzlich mindestens einen Vernetzer enthält.

12. Kautschukmischung gemäß einem der Ansprüche 9 bis 11, **dadurch gekennzeichnet, dass** sie als Kautschuk mindestens einen SBR-Kautschuk und mindestens einen BR-Kautschuk, vorzugsweise im Gewichtsverhältnis SBR-Kautschuk: BR-Kautschuk von 60:40 bis 90:10 enthält.

13. Kautschukmischung gemäß Anspruch 12, **dadurch gekennzeichnet, dass** sie zusätzlich mindestens einen NR-Kautschuk enthält, vorzugsweise in einem Gewichtsverhältnis SBR-Kautschuk zu BR-Kautschuk zu NR-Kautschuk von 60 bis 85 : 10 bis 35 : 5 bis 20.

14. Verfahren zur Herstellung von Kautschukmischungen gemäß einem der Ansprüche 9 bis 13, durch Mischen von mindestens jeweils einem Kautschuk und einer Polysulfidmischung gemäß einem der Ansprüche 1 bis 3 oder Polysulfidverbindungen gemäß Anspruch 8.

15. Verfahren zur Herstellung von Vulkanisaten **dadurch gekennzeichnet, dass** man die Kautschukmischung gemäß einem der Ansprüche 9 bis 13 vulkanisiert, vorzugsweise bei Massetemperaturen von 100 bis 200 °C, besonders bevorzugt von 130 bis 180 °C.

16. Vulkanisate, erhältlich durch Vulkanisation der Kautschukmischungen gemäß einem der Ansprüche 9 bis 13.

17. Kautschukprodukte, insbesondere Reifen enthaltend ein oder mehrere Kautschukvulkanisate gemäß Anspruch 16.

18. Fahrzeug enthaltend Kautschukprodukt gemäß Anspruch 17.

19. Verwendung von Polysulfidmischungen gemäß einem der Ansprüche 1 bis 3 oder Polysulfidverbindungen gemäß Anspruch 8 zur Herstellung von Kautschukmischungen und deren Vulkanisaten.

20. Additivzusammensetzung enthaltend jeweils mindestens ein schwelfelhaltiges Alkoxysilan, insbesondere Bis-(triethoxysilylpropyl)tetrasulfan, Bis-(triethoxysilyl-propyl)disulfan, 3-(Triethoxysilyl)-1-propanthiol, polyetherfunktionalisiertes Mercaptosilan oder thioesterfunktionalisiertes Alkoxysilan und eine Polysulfidmischung gemäß einem der Ansprüche 1 bis 3 oder Polysulfidverbindungen gemäß Anspruch 8.

21. Verwendung von Polysulfidmischungen gemäß einem der Ansprüche 1 bis 3 und/oder Polysulfidverbindungen gemäß Anspruch 8 zur Herstellung von Additivzusammensetzungen gemäß Anspruch 20.

22. Verfahren zur Verringerung des Rollwiderstands von Reifen, **dadurch gekennzeichnet, dass** man einer nicht- oder teilvernetzten Kautschukmischung, welche als Ausgangsmaterial für zumindest Teile des Reifen dient, eine Polysulfidmischungen gemäß einem der Ansprüche 1 bis 3 oder Polysulfidverbindungen gemäß Anspruch 8 beimengt und die Mischung anschließend vulkanisiert.

## Claims

1. Polysulfide mixtures comprising two or more compounds of the formula (I), 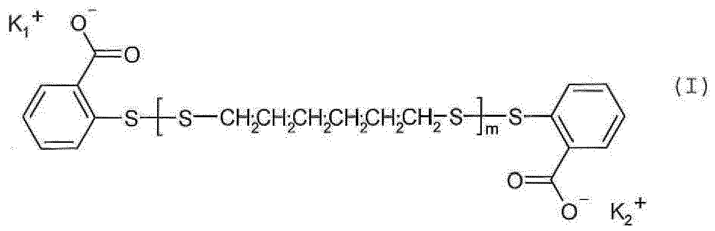 where K₁⁺ and K₂⁺ are identical or different, preferably identical, and mutually independently are any desired monovalent cation or the nth part of any desired n-valent cation, and
m is 0, 1 and/or 2,
**characterized in that** the total proportion of compound(s) where m = 1 is at least 80%, preferably at least 85%, particularly preferably at least 90%, very particularly preferably from 93 to 99%, based on the total quantity of polysulfide compounds of the formula (I) where m = 0, 1, and 2.

2. Polysulfide mixture according to Claim 1, **characterized in that** K₁⁺ and K₂⁺ are identical or different, preferably identical, and are mutually independently H⁺, an alkali metal cation, in particular Li⁺, Na⁺, K⁺, ½ alkaline earth metal cation, in particular ½ Mg²⁺, ½ Ca²⁺, 1/3 Al³⁺, are the nth part of an n-valent rare earth metal cation, or are ½ Zn²⁺, particularly preferably are H⁺ or ½ Zn²⁺.

3. Polysulfiden mixture according to Claim 1 or 2, **characterized in that** its total chlorine content is < 1%, preferably < 0.1%, particularly preferably < 200 ppm, very particularly preferably < 50 ppm, most preferably < 10 ppm.

4. Process for the production of a polysulfide mixture according to any of Claims 1 to 3, **characterized in that** one or more compounds of the formula (III) 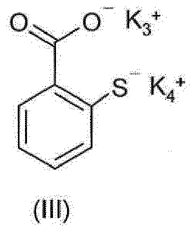 where cations K₃⁺ and K₄⁺ are mutually independently any desired monovalent cations or are the nth part of any desired n-valent cation, preferably being H⁺, are an alkali metal cation, in particular Li⁺, Na⁺, K⁺, ½ alkaline earth metal cation, in particular ½ Mg²⁺, ½ Ca²⁺, 1/3 Al³⁺, the nth part of an n-valent rare earth metal cation, or are ½ Zn²⁺, particularly preferably being H⁺, Na⁺, K⁺ or ½ Zn²⁺,
is/ are brought into contact with one or more compounds of the formula (IV), 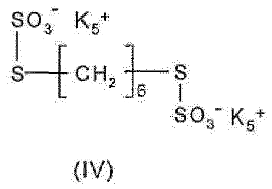 where the two cations K₅⁺ here are identical or different, preferably identical, and mutually independently are any desired monovalent cations or are the nth part of any desired n-valent cation, preferably being an alkali metal cation, in particular Li⁺, Na⁺, K⁺, ½ alkaline earth metal cation, in particular ½ Mg²⁺, ½ Ca²⁺, 1/3 Al³⁺, are the nth part of an n-valent rare earth metal cation, or are ½ Zn²⁺, particularly preferably being Na⁺,
**characterized in that** the contact is effected at temperatures in the range from -5°C to 19°C, preferably from 0°C to 15°C, particularly preferably from 0°C to 10°C.

5. Process according to Claim 4, **characterized in that** compound(s) of the formula (III) where K₃⁺ and/or K₄⁺ ≠ H⁺ are added and the pH of the reaction mixture is kept in the range from pH 7 to 13, preferably from 8 to 12, in particular from 9 to 11, preferably via addition of a Bronsted acid.

6. Process according to Claim 4 or 5, **characterized in that**, after contact of the compound of the formula (III) with at least one compound of the formula (IV), the Polysulfide mixture is precipitated via addition of at least one acid, in particular hydrochloric acid or sulfuric acid, and/or contact with at least one salt of the cations K₁⁺ and/or K₂⁺, where K₁⁺ and/or K₂⁺ is/are a cation other than H⁺.

7. Process according to one of Claims 4 to 6, **characterized in that**, after contact of the compound of the formula (III) with at least one compound of the formula (IV), the polysulfide mixture is precipitated via contact of at least one salt of the cation K₁⁺ and/or K₂⁺, where K₁⁺ and/or K₂⁺ is/are a cation other than H⁺, preferably a zinc salt, in particular zinc sulfate.

8. Polysulfide compounds of the formula (I) according to Claim 1, where K₁⁺ + and K₂⁺ are identical or different, preferably identical, and mutually independently are any desired monovalent cation or are the nth part of any desired n-valent cation, preferably being H⁺, an alkali metal cation, in particular Li⁺, Na⁺, K⁺, ½ alkaline earth metal cation, in particular ½ Mg²⁺, ½ Ca²⁺, 1/3 Al³⁺, are the nth part of an n-valent rare earth metal cation, or are ½ Zn²⁺, particularly preferably being ½ Zn²⁺,
where K₁⁺ and/or K₂⁺, preferably K₁⁺ and K₂⁺, is/are a cation other than H⁺, and
m is 1 or 2, preferably 1.

9. Rubber mixture comprising respectively at least
- one rubber
- one polysulfide mixture according to any of Claims 1 to 3, or polysulfide compounds according to Claim 8.

10. Rubber mixture according to Claim 9 also comprising at least
- one sulfur-containing Alkoxysilane, and
- one silica-based filler.

11. Rubber mixture according to Claim 9 or 10, **characterized in that** it also comprises at least one crosslinking agent.

12. Rubber mixture according to any of Claims 9 to 11, **characterized in that** it comprises, as rubber, at least one SBR and at least one BR, where the ratio by weight of SBR:BR is preferably from 60:40 to 90:10.

13. Rubber mixture according to Claim 12, **characterized in that** it also comprises at least one NR, where the ratio by weight of SBR to BR to NR is preferably from 60 to 85 : from 10 to 35 : from 5 to 20.

14. Process for the production of rubber mixtures according to any of Claims 9 to 13 via mixing of at least respectively one rubber and one polysulfide mixture according to any of Claims 1 to 3 or polysulfide compounds according to Claim 8.

15. Process for the production of vulcanizates, **characterized in that** the rubber mixture according to any of Claims 9 to 13 is vulcanized, and the temperatures of the composition are preferably from 100 to 200°C, particularly preferably from 130 to 180°C.

16. Vulcanizates obtainable via vulcanization of the rubber mixtures according to any of Claims 9 to 13.

17. Rubber products, in particular tires comprising one or more rubber vulcanizates according to Claim 16.

18. Vehicle comprising rubber product according to Claim 17.

19. Use of polysulfide mixtures according to any of Claims 1 to 3 or Polysulfide compounds according to Claim 8 for the production of rubber mixtures and vulcanizates thereof.

20. Additive composition comprising respectively at least one sulfur-containing alkoxysilane, in particular bis(triethoxysilylpropyl) tetrasulfane, bis(triethoxysilylpropyl) disulfane, 3-(triethoxysilyl)-1-propanethiol, polyether-functionalized mercaptosilane, or thioester-functionalized alkoxysilane, and a polysulfide mixture according to any of Claims 1 to 3 or polysulfide compounds according to Claim 8.

21. Use of polysulfide mixtures according to any of Claims 1 to 3 and/or Polysulfide compounds according to Claim 8 for the production of additive compositions according to Claim 20.

22. Process for reducing the rolling resistance of tires, **characterized in that** polysulfide mixtures according to any of Claims 1 to 3 or Polysulfide compounds according to Claim 8 are mixed with a non-crosslinked or partially crosslinked rubber mixture serving as starting material for at least parts of the tire, and the mixture is then vulcanized.

## Revendications

1. Mélange de polysulfures contenant deux composés de formule (I) ou plus 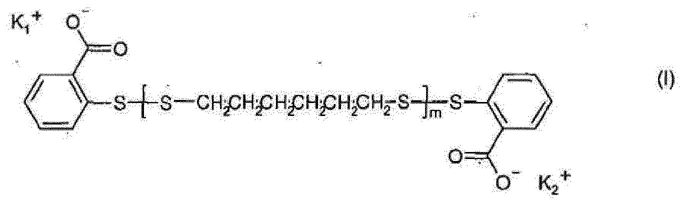 K₁⁺ et K₂⁺ étant identiques ou différents, de préférence identiques et représentant, indépendamment l'un de l'autre, un cation monovalent quelconque ou la n^{ieme} partie d'un cation n-valent quelconque, et
m représentant 0, 1 et/ou 2,
**caractérisé en ce que** la proportion totale de composé(s) présentant m = 1 est d'au moins 80%, de préférence d'au moins 85%, de manière particulièrement préférée d'au moins 90%, de manière tout particulièrement préférée de 93-99%, par rapport à la quantité totale de composés de type polysulfure de formule (I) avec m = 0, 1 et 2.

2. Mélange de polysulfures selon la revendication 1, **caractérisé en ce que** K₁⁺ et K₂⁺ sont identiques ou différents, de préférence identiques et représentent, indépendamment l'un de l'autre, H⁺, un cation de métal alcalin, en particulier Li⁺, Na⁺, K⁺, 1/2 cation de métal alcalino-terreux, en particulier 1/2 Mg²⁺, 1/2 Ca²⁺, 1/3 Al³⁺, la n^{ième} partie d'un cation n-valent de métal des terres rares ou 1/2 Zn²⁺, de manière particulièrement préférée H⁺ ou 1/2 Zn²⁺.

3. Mélange de polysulfures selon la revendication 1 ou 2, **caractérisé en ce qu'**il présente une teneur totale en chlore < 1%, de préférence < 0,1%, de manière particulièrement préférée < 200 ppm, de manière tout particulièrement préférée < 50 ppm, de manière tout à fait préférée < 10 ppm.

4. Procédé pour la préparation d'un mélange de polysulfures selon l'une quelconque des revendications 1 à 3, **caractérisé en ce qu'**on met en contact un ou plusieurs composés de formule (III) 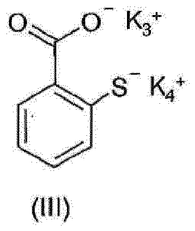 les cations K₃⁺ et K₄⁺ représentant, indépendamment l'un de l'autre, des cations monovalents quelconques ou la n^{ième} partie d'un cation n-valent quelconque, de préférence H⁺, un cation de métal alcalin, en particulier Li⁺, Na⁺, K⁺, 1/2 cation de métal alcalino-terreux, en particulier 1/2 Mg²⁺, 1/2 Ca²⁺, 1/3 Al³⁺, la n^{ième} partie d'un cation n-valent de métal des terres rares ou 1/2 Zn²⁺, de manière particulièrement préférée H⁺, Na⁺, K⁺ ou 1/2 Zn²⁺,
avec un ou plusieurs composés de formule (IV) 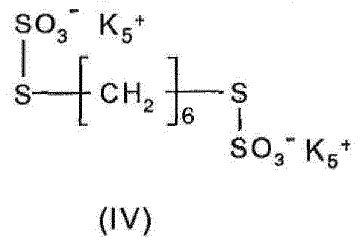 les deux cations K₅⁺ étant identiques ou différents, de préférence identiques et représentant, indépendamment l'un de l'autre, des cations monovalents quelconques ou la n^{ième} partie d'un cation n-valent quelconque, de préférence un cation de métal alcalin, en particulier Li⁺, Na⁺, K⁺, 1/2 cation de métal alcalino-terreux, en particulier 1/2 Mg²⁺, 1/2 Ca²⁺, 1/3 Al³⁺, la n^{ième} partie d'un cation n-valent de métal des terres rares ou 1/2 Zn²⁺, de manière particulièrement préférée Na⁺, **caractérisé en ce que** la mise en contact est réalisée à des températures dans la plage de -5°C à 19°C, de préférence de 0°C à 15°C, de manière particulièrement préférée de 0°C à 10°C.

5. Procédé selon la revendication 4, **caractérisé en ce qu'**un/des composé(s) de formule (III) avec K₃⁺ et/ou K₄⁺ ≠ H⁺ sont ajoutés et le pH du mélange réactionnel est maintenu dans la plage de pH 7-13, de préférence de 8-12, en particulier de 9-11, de préférence par addition d'un acide de Brönsted.

6. Procédé selon la revendication 4 ou 5, **caractérisé en ce qu'**après la mise en contact du composé de formule (III) avec au moins un composé de formule (IV), le mélange de polysulfures est précipité par addition d'au moins un acide, en particulier de l'acide chlorhydrique ou de l'acide sulfurique et/ou par mise en contact avec au moins un sel des cations K₁⁺ et/ou K₂⁺, K₁⁺ et/ou K₂⁺ représentant un cation différent de H⁺.

7. Procédé selon l'une quelconque des revendications 4 à 6, **caractérisé en ce qu'**après la mise en contact du composé de formule (III) avec au moins un composé de formule (IV), le mélange de polysulfures est précipité par mise en contact avec au moins un sel des cations K₁⁺ et/ou K₂⁺, K₁⁺ et/ou K₂⁺ représentant un cation différent de H⁺, de préférence un sel de zinc, en particulier un sulfate de zinc.

8. Composés de type polysulfure de formule (I) selon la revendication 1, K₁⁺ et K₂⁺ étant identiques ou différents, de préférence identiques et représentant, indépendamment l'un de l'autre, un cation monovalent quelconque ou la n^{ième} partie d'un cation n-valent quelconque, de préférence H⁺, un cation de métal alcalin, en particulier Li⁺, Na⁺, K⁺, 1/2 cation de métal alcalino-terreux, en particulier 1/2 Mg²⁺, 1/2 Ca²⁺, 1/3 Al³⁺, la n^{ième} partie d'un cation n-valent de métal des terres rares ou 1/2 Zn²⁺, de manière particulièrement préférée 1/2 Zn²⁺,
K₁⁺ et/ou K₂⁺, de préférence K₁⁺ et K₂⁺, représentant un cation différent de H⁺, et
m représentant 1 ou 2, de préférence 1.

9. Mélange pour caoutchouc contenant à chaque fois au moins
- un caoutchouc
- un mélange de polysulfures selon l'une quelconque des revendications 1 à 3 ou des composés de type polysulfure selon la revendication 8.

10. Mélange pour caoutchouc selon la revendication 9, contenant en outre au moins
- un alcoxysilane contenant du soufre et
- une charge à base de silice.

11. Mélange pour caoutchouc selon la revendication 9 ou 10, **caractérisé en ce qu'**il contient en outre au moins un réticulant.

12. Mélange pour caoutchouc selon l'une quelconque des revendications 9 à 11, **caractérisé en ce qu'**il contient, comme caoutchouc, au moins un caoutchouc SBR et au moins un caoutchouc BR, de préférence dans un rapport pondéral caoutchouc SBR:caoutchouc BR de 60:40 à 90:10.

13. Mélange pour caoutchouc selon la revendication 12, **caractérisé en ce qu'**il contient en outre au moins un caoutchouc NR, de préférence dans un rapport pondéral caoutchouc SBR:caoutchouc BR:caoutchouc NR de 60-85:10-35:5-20.

14. Procédé pour la préparation de mélanges pour caoutchouc selon l'une quelconque des revendications 9 à 13, par mélange d'au moins un caoutchouc et d'un mélange de polysulfures selon l'une quelconque des revendications 1 à 3 ou de composés de type polysulfure selon la revendication 8.

15. Procédé pour la préparation de produits vulcanisés, **caractérisé en ce qu'**on vulcanise le mélange pour caoutchouc selon l'une quelconque des revendications 9 à 13, de préférence à des températures de la masse de 100 à 200°C, de manière particulièrement préférée de 130 à 180°C.

16. Produits vulcanisés, pouvant être obtenus par vulcanisation des mélanges pour caoutchouc selon l'une quelconque des revendications 9 à 13.

17. Produits en caoutchouc, en particulier pneus contenant un ou plusieurs produits vulcanisés de caoutchouc selon la revendication 16.

18. Véhicule contenant le produit de caoutchouc selon la revendication 17.

19. Utilisation de mélanges de polysulfures selon l'une quelconque des revendications 1 à 3 ou de composés de type polysulfure selon la revendication 8 pour la préparation de mélanges pour caoutchouc et leurs produits de vulcanisation.

20. Composition d'additif, contenant à chaque fois au moins un alcoxysilane contenant du soufre, en particulier le bis-(triéthoxysilylpropyl)tétrasulfane, le bis-(triéthoxysilylpropyl)disulfane, le 3-(triéthoxysilyl)-1-propanethiol, un mercaptosilane fonctionnalisé par polyéther ou un alcoxysilane fonctionnalisé par thioester et un mélange de polysulfures selon l'une quelconque des revendications 1 à 3 ou un composé de type polysulfure selon la revendication 8.

21. Utilisation de mélanges de polysulfures selon l'une quelconque des revendications 1 à 3 et/ou de composés de type polysulfure selon la revendication 8 pour la préparation de compositions d'additif selon la revendication 20.

22. Procédé pour la diminution de la résistance au roulement de pneus, **caractérisé en ce qu'**on mélange dans un mélange pour caoutchouc non réticulé ou partiellement réticulé, qui sert de matériau de départ pour au moins des parties du pneu, un mélange de polysulfures selon l'une quelconque des revendications 1 à 3 ou des composés de type polysulfure selon la revendication 8 et on vulcanise ensuite le mélange.
